# EUROPEAN PATENT APPLICATION

(11) **EP 4 156 096 A1**
(43) Date of publication of application: **29.03.2023**
(21) Application number: 22190450.1
(22) Date of filing: 16.08.2022
(51) Int. Cl.: G06T 7/00

(54) **METHOD, DEVICE AND SYSTEM FOR AUTOMATED PROCESSING OF MEDICAL IMAGES TO OUTPUT ALERTS FOR DETECTED DISSIMILARITIES**

(30) Priority: 23.09.2021 EP 21198541
(71) Applicant: Siemens Healthcare GmbH, 91052 Erlangen (DE)
(72) Inventor: Shinagawa, Yoshihisa, Downingtown, 19335 (US)

(57) **Abstract**

A method, device and system for automated processing of medical images to output alerts (A) for detected dissimilarities in the medical images is provided. In one aspect, the method comprises: receiving (301, 401) a first medical image of an anatomical object of a patient, the first medical image being acquired at a first instance of time, receiving (302, 402) a second medical image of the anatomical object of the patient, the second medical image being acquired at a second instance of time, determining (303, 405) a dissimilarity between the received first medical image and the received second medical image using a trained machine learning network, the trained machine learning network being adapted to determine two medical images having differences besides posture deformation, motion, variations of anatomy and scanning parameters as dissimilar and two medical images having no difference or only differences due to posture deformation, motion, variations of anatomy and scanning parameters as similar, and outputting (304, 406) a third medical image being generated based on the received first medical image and/or the received second medical image, said third medical image including an alert (A) for each determined dissimilarity, said alert (A) visualizing the determined dissimilarity between the received first medical image and the received second medical image in the third medical image.

## Description

The present invention relates to a computer-implemented method, to a computer-implemented device and to a system for automated processing of medical images to output alerts for detected dissimilarities in the medical images.

For detecting dissimilarities in medical images, for example for detecting a change in medical images of a pair of prior and current chest volumes, radiologists routinely read follow-up cases, e.g., a sequence of medical images of a certain anatomical object of a patient captured over a certain time period, for example two years. The radiologists compare prior studies and a current study, by comparing the corresponding medical images, to report interval changes and provide a new diagnosis, if any.

A first disadvantage of this conventional method is that it is cumbersome to compare two sets of images side by side, with one coming from the prior study and the other one from the current study, because the radiologists have to move their eye focus frequently between them. A second disadvantage is that doing so has not a very high accuracy in detecting dissimilarities, for example changes in the lung of a patient. A third disadvantage is that this conventional method is very time -consuming.

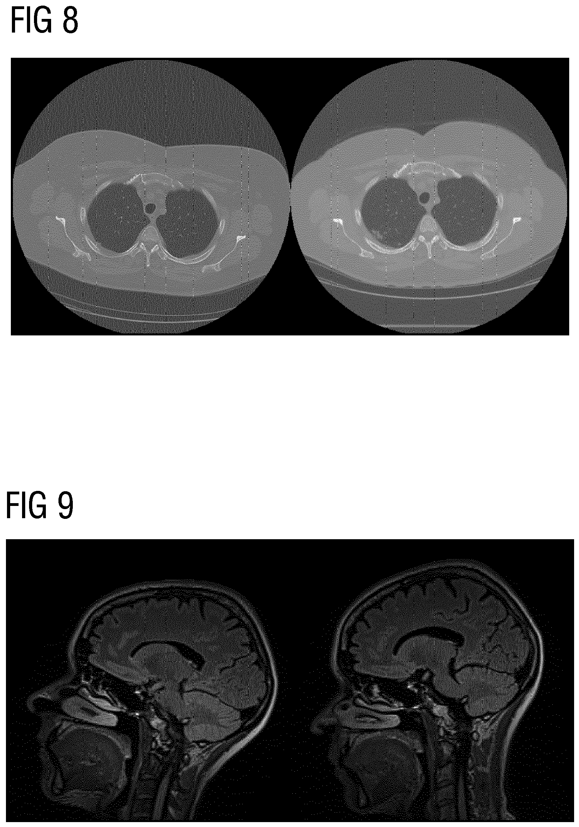

In this regard, FIG. 8 shows an example of conventional medical images showing a set of corresponding slices of a pair of prior and current chest volumes. For example, there is new nodule appearing in the right lung as shown in FIG. 8. Often times, due to the differences of postures and motions of organs, changes are not obvious to radiologists. Radiologists need to carefully compare in order not to miss changes. For example, one of the changes as shown in FIG. 9 around the sella turcica is not easily noticed due to the difference in the posture of the head.

Therefore, there is a need for a method and device which enables accurate and fast detection of dissimilarities in medical images.

There are conventional techniques for detecting changes between volumes by first registering the two volumes and then subtracting the prior from the current, i.e., the two volumes are brought to the same posture by applying rigid or deformable transformations to them and subtract them. These conventional techniques are useful to detect subtle changes and analyze the changes in detail, such as the growth of tumors. However, these conventional techniques are susceptible to noise and motion such as inspiration and/or expiration, and also scanning parameter differences. Too often, too many changes are detected.

Examples for these conventional techniques are disclosed by documents US 2014/0294263 A1, US 6678399 B2, and US 2005/0165292 A1. In detail, US 2014/0294263 A1 describes a framework for facilitating synchronized image navigation. At least first and second medical images are received. A non-linear mapping between the first and second medical images is generated. A selection of a given location in the first medical image is received in response to a user's navigational operation. Without deforming the second medical image, a target location in the second medical image is determined by using the non-linear mapping. The target location corresponds to the given location in the first medical image. An optimized deformation-free view of the second medical image is generated based at least in part on the target location. While the user performs navigational operations on the first medical image, the framework repeatedly receives the selection of the given location, determines the target location using the non-linear mapping, and generates the optimized deformation-free view of the second medical image based at least in part on the target location.

Further, US 6678399 B2 describes a method for computerized detection of lung nodules in computer tomography images, by which mask images are created such that subtractions of the mask image from a targeted CT section image reveal or highlight small lung nodules in the target CT section. The mask image is created utilizing the targeted CT section image along with other CT section images generated from the same CT scan. Based on these other section CT images and the targeted CT section image, a mask image can be created that is very similar to the target CT section image, but without the presence of small lung nodules. When the mask image is subtracted from the targeted CT section image, the differences between the mask images and the CT section images reveal small lung nodules. The mask image may be created by linear interpolation or a morphological filtered image.

Moreover, US 20050165292 A1 describes a method and an apparatus for allowing determination of patient position change relative to an imaging device and/or allowing digital subtraction in an operative position. The apparatus can include devices for determining a position of a patient at various times and comparing the various positions of the patient. Further, a digital subtraction may be performed if the patient change is not above a threshold value and/or if motion correction can occur.

EP 3 444 777 A1 proposes a method focusing on the generation of an optimized deformation field between the prior and the follow-up image. In particular, it is proposed to use a trained function to derive a deformation field which differentiates between physiological changes (due to "healthy" tissue changes based, e.g., on breathing, different placements of a patient in the underlying imaging procedure, weight gain or loss, etc.) and pathological changes requiring the attention of the user.

One issue even with sophisticated ways to calculate an adapted deformation field is that such methods are still prone to produce artefacts. This is because tissue in general has very heterogenous deformation properties the prediction of which is difficult even with machine learned functions.

The object of the invention is therefore to provide a method, device and system that enables an improved detection of dissimilarities in medical images.

According to an aspect, a computer-implemented method for automated processing of medical images to output alerts for detected dissimilarities in the medical images is provided. The method comprises:
- receiving a first medical image of an anatomical object of a patient, the first medical image being acquired at a first instance of time,
- receiving a second medical image of the anatomical object of the patient, the second medical image being acquired at a second instance of time,
- determining an image similarity between image data of the first medical image and image data of the second medical image,
- determining a dissimilarity between the first medical image and the second medical image based on the image similarity, and
   outputting an alert for the dissimilarity (e.g., in a user interface).

In other words, dissimilarities are identified based on the evaluation of image similarities and not, as in methods according to the prior art, based on calculating a registration and corresponding deformation fields. This makes the identification of dissimilarities, or in other words pathologically relevant changes, less prone to errors and more reliable. In this regard, the inventor recognized that pathological relevant changes such as newly occurred or vanished lesions can be detected by searching for of image data which is dissimilar in the prior image as compared to the follow-up image.

Determining image similarities may mean determining, for pairs of image data in the first and second medical image, a similarity metric representing the image similarity between image data that is being compared. Based on this evaluation, spots having the greatest dissimilarity may be selected and identified. For instance, all locations having a similarity below a predetermined threshold may be selected. The result of determining a similarity metric between image data may be a degree of (image) similarity.

The medical images may be captured by and received from a medical imaging unit. The medical imaging unit may include, for example, but not limited to, magnetic resonance imaging device, computed tomography device, X-ray imaging device, ultrasound imaging device, etc. The medical images may be three-dimensional and/or related to a volume. Further, the medical images may be two-dimensional. The medical images may include one or more anatomical objects associated with a patient. The anatomical objects may be in particular one or more body parts associated with the patient that may have been imaged. The anatomical objects may include, but not be limited to, one or more imaged organs, such as the lung. The medical images may, for example, be in the form of an array of pixels or voxels. Such arrays of pixels or voxels may be representative of intensity, absorption or other parameter as a function of three-dimensional position, and may, for example, be obtained by suitable processing of measurement signals obtained by one or more of the above-mentioned medical imaging units. In particular, the medical image may include information associated with a region of interest for tumor detection. Alternatively, the medical images may be received from a medical database including a plurality of medical images.

According to an aspect, the step of outputting comprises outputting (optionally via the user interface) a third medical image being generated based on the received first medical image and/or the received second medical image, said third medical image including an alert for each determined dissimilarity, said alert visualizing the determined dissimilarity between the received first medical image and the received second medical image in the third medical image.

With that, the dissimilarities and the alert are shown in relation to the respective location in the first and/or second image. Accordingly, the user is provided with an actionable readout which she or he can immediately verify and evaluate.

According to an aspect, the step of determining the dissimilarity comprises comparing the image similarity with a predetermined threshold.

In other words, the comparison to a predetermined threshold allows to systematically identify portions of first and second images which are so dissimilar that they could point to a pathological change requiring the attention of a physician. For instance, any image data in the first and/or second medical image having an image similarity (or an image similarity value) below the predetermined threshold may be identified as a dissimilarity.

According to an aspect, the method comprises
- receiving a user input directed to set the pre-determined threshold, and
- setting the pre-determined threshold based on the user input.

With that, the threshold and, thus, the sensitivity of the dissimilarity detection may be interactively set by the user. In particular, the user input directed to set the pre-determined threshold may be received after outputting the dissimilarity and the method may further comprise adapting the outputting based on the step of setting the pre-determined threshold.

According to an aspect, the step of determining the image similarity comprises:
- obtaining a first feature signature from the first medical image based on first image data of the first medical image,
- obtaining a second feature signature from the second medical image based on second image data of the second medical image, and
- calculating a similarity signature (or an image similarity value) based on the first feature signature and the second feature signature, the similarity signature indicating (or quantifying) the image similarity between first and second image data.

A feature signature generally may be conceived as a descriptor representing or characterizing the underlying medical image. A feature signature may comprise a plurality of individual feature vectors relating to different locations of the medical image (see below). A feature signature as herein described may be based on image data of the medical images.

In some examples, a feature signature and/or individual feature vectors may be determined by encoding image data associated with the respective medical image using a particular encoding algorithm (also referred to as "vectorizer)". In particular, such an algorithm may be a trained machine learning algorithm. Features of a feature signature or feature vector may be visual features, such as a visual manifestation of a medical abnormality, a pattern, an anatomy, a medical landmark and so forth as indicated by the image data of the respective medical image. Feature signatures or vectors may be calculated on-line, e.g., upon receiving the medical image. Alternatively, feature signatures or vectors may be held available as -pre-generated data items stored in a database, e.g., in association with the medical images.

According to some examples, the image similarity may be quantified in terms of the similarity signature by applying a metric ("image similarity metric") representing how similar or dissimilar image data of two different medical images is. Another expression for image similarity metric may be similarity metric or distance metric. In some examples, the image similarity metric may be configured to quantify a distance in vector or feature space between first and second feature signatures. According to some examples, predetermined mathematical functions may be used to calculate such a distance such as the cosine similarity or the Euclidean distance and so forth. According to other examples, the image similarity metric may comprise a learned metric which has been derived by machine learning. According to some implementations, the extraction of the feature signatures or vectors and/or the evaluation of a similarity metric as a whole may be performed by one or more trained machine learning algorithm.

The similarity signature may comprise integers, floating point numbers or even Boolean variables (e.g., similarity present vs. similarity not present). The similarity signature may comprise a plurality of individual similarity values or degrees of similarity. Thereby, the individual similarity values may be locally resolved according to the image space of first and/or second medical images (see below). In the step of outputting, these values may be translated or converted into a representation that can be readily assessed by the human eye, for instance. In other words, a visualization (a viewable representation) may be generated (calculated) based on the calculated similarity signature. To this end, individual values comprised in the similarity signature may be normalized, e.g., with respect to the highest degree of similarity calculated. The step of outputting may further comprise a step of interpolating between individual degrees of similarity in order to get a larger data base for visualization. Further, the step of outputting may comprise a step of smoothing the degrees of similarity in order to allow for a more homogenous visualization.

According to an aspect, the step of determining the first feature signature comprises:
- defining a plurality of first (image) patches in the first medical image,
- obtaining for each of the first patches a first feature vector based on the image data of the respective patch, wherein the first feature signature includes the plurality of first feature vectors,
- the step of determining the second feature signature comprises:
- defining a plurality of second (image) patches in the second medical image, the second patches corresponding to the first patches,
- obtaining, for each of the second patches a second feature vector, wherein the second feature signature includes the plurality of second feature vectors,
- the step of calculating the similarity signature comprises, for each of the first patches,
- calculating a local similarity based on the first feature vector of the respective first patch and the second feature vector of the corresponding second patch, wherein the local similarity indicates a degree of similarity between the image data of the respective first image patch and the image data of the corresponding second image patch.

In other words, first and second medical images are respectively partitioned in one or more image patches to allow for a spatially resolved readout of the image similarities and a localized identification of any dissimilarities.

To this end, a plurality of two-dimensional image patches is defined in the medical images and feature vectors are respectively generated for each patch. In particular, first and second image patches may be defined such that each of a plurality of first patches corresponds or conforms to a second patch. In particular, corresponding may mean that first and second image patches are located at substantially the same locations in the first and second medical images, respectively. In other words, first and second image patches may be defined such that pairs of corresponding first and second image patches result. Based on the first and second feature vectors associated with the respective first and second image patches, a degree of similarity (or local similarity) can be calculated for each pair of first and second image patch. To do so, the respective feature vectors of corresponding first and second image patches may be compared to each other. The local (degree of) similarity is indicative of the local image similarity of the image data respectively comprised in corresponding first and second image patches. Thereby, the feature vectors may be extracted essentially as described before. In particular, generating the feature vectors and, optionally, determine local similarities may comprise inputting first and second image patches into a trained machine learning algorithm.

Since the image patches preferably span the medical images, the same holds true for the local similarities - as they are calculated for each pair of image patches.

Accordingly, the local similarities comprised in the similarity signature give information about local dissimilarities which, in turn, could hint at pathological changes a user should review. The individual local similarities are a measure for the (image-)similarity of the respective pair of image patches. High values for the local similarity may indicate a high similarity whereas low values stand for a dissimilarity.

Of note, the step of defining the image patches and the step of calculating the feature vectors do not need to be performed sequentially in the sense that first all image patches are defined (and stored) and then the degrees of similarity are calculated. Rather, the degree of similarity of a given pair of image patches may be calculated once the image patches have been defined. This has the benefit that the image patches as such do not have to be buffered and only the local similarities are recorded. Such procedure is explicitly included in the scope of the claims since this still involves the definition of the image patches at some point.

According to an aspect, the step of determining the dissimilarity comprises identifying dissimilar pairs of first and second image patches based on the local similarities.

According to an aspect, the step of outputting comprises outputting an alert for each identified dissimilar pair of first and second image patches.

According to an aspect, the step of outputting comprises including an alert for each identified dissimilar pair of first and second image patches, said alert visualizing the determined dissimilar patches in the third medical image.

In other words, an alert is allocated to a location of the first and/or second patch of a dissimilar pair of first and second image patches. With that, a spatially resolved and actionable output can be generated for the user.

According to an aspect, the steps of defining first and/or second image patches is performed using a sliding window method or an overlapping sliding window method.

In the sliding window approach, a window "slides" over an image (e.g., the representation image and/or the first/second slices and/or the sequential images) for dividing it into patches. Every slide, the window is moved a specific number of pixels to the side, which is also called "the stride". The stride may be such that subsequent image patches may comprise some overlap with the previous image patches. Preferably, the size of the window is adjusted such that the window has approximately the same size in the first and second medical image.

The usage of a sliding window algorithm for defining the patches has the advantage, that the image patches can be sequentially defined in the sense that a new image patch may be only defined once the degree of similarity of the preceding patch has been calculated. Moreover, the sliding window method is efficient form a computational point of view. The use of an overlapping sliding window method enables to more readily capture individual structures in the medical images. This is because it is usually unlikely that patterns in an image conform to a fixed grid of image patches. This might have the consequences that patterns may be cut by the image patches adversely affecting the calculation result (i.e., the local similarity). By using an overlapping sliding window approach, a form of a mean calculation is applied across a plurality of image patches partially mitigating such finite size effects. Moreover, the resolution of the degrees of similarity with respect to the medical image volume is enhanced.

As an alternative, first and/or second image patches may be defined by applying a grid to the representation image and/or the second slices and/or the sequential images which likewise allows for a computationally fast and scalable way of partitioning the images into image patches. Preferably, the size of the grid is the same for first and second medical image.

According to an aspect, the method further comprises:
obtaining an image registration between the first medical image and the second medical image, wherein
the step of determining the image similarity between image data of the first medical image and image data of the second medical image is based on the image registration.

In particular, the step of defining the plurality of first (image) patches in the first medical image, and/or the step of defining the plurality of second (image) patches in the second medical image, may be based on the image registration.

Determining at least one image registration, according to some examples, may in general comprise registering a target image (e.g., the first image) with a reference image of a time series (e.g., the second image). According to some examples, this may comprise obtaining a deformation field between target and reference image that determines a relationship between the coordinate systems of the target image data and the reference image data such that each anatomical location in the target image is mapped to roughly the same anatomical location in the reference image and vice versa. Thus, the deformation field may comprise a plurality of individual displacement vectors respectively associated with the pixels/voxels of the target image and the reference image.

According to some examples, the registration may comprise a rigid registration. A rigid registration may comprise a registration in which the coordinates of pixels/voxels in one image are subject to rotation and translation in order to register the image to another image. According to some examples, the registration may comprise and affine registration. An affine registration may comprise a registration in which the coordinates of data points in one image are subject to rotation, translation, scaling and/or shearing in order to register the image to another image. Thus, a rigid registration may be considered to be a particular type of affine registration. According to some examples, the registration may comprise a non-rigid registration. A non-rigid registration may provide different displacements for each pixel/voxel of the image to be registered and can, for example, use non-linear transformations, in which the coordinates of pixels/voxels in one image are subject to flexible deformations in order to register the image to another image. Non-linear transformations may, according to some examples, be defined using vector fields such as warp fields, or other fields or functions, defining an individual displacement for each pixel/voxel in an image. For more detailed information about image registration, reference is made to US 2011 / 0 081 066 and US 2012 / 0 235 679. Rigid image registration is very effective in cases when no anatomic change or deformations are expected. In comparison to rigid image registration, non-rigid image registration has a significantly greater flexibility as non-rigid image registrations can manage local distortions between two image sets (e.g. anatomical structure changes) but can be more complex to handle.

By calculating an image registration und considering the image registration upon calculating the image similarities and, in particular, upon defining the image patches, potential global misalignments or offsets between the first and second medical images can be compensated for.

Since the actual recognition of dissimilarities is still based on calculating image similarities and not on a direct evaluation of the image registration, the registration may be comparably coarse thereby avoiding the usual artefacts arising from change assessments based on image registrations. In particular, rigid registrations may already be sufficient for improving the ensuing dissimilarity assessment based on calculating image similarities.

According to an aspect, the method further comprises
extracting a first slice depicting a certain section of the anatomical object from the first medical image,
extracting a second slice depicting the certain section of the anatomical object from the second medical image,
wherein the image similarity is an image similarity between image data of the first slice and image data of the second slice.

According to the above aspect, processing of three-dimensional medical images is more readily enabled. Firstly, slices are extracted that shall be investigated for dissimilarities. Secondly, the methodology for the image similarity calculation as introduced above (including the optional calculation of the similarity signature and/or the local similarities) may be applied to the extracted first and second slices.

According to an aspect, the first feature signature may be obtained based on first image data of the first slice, and the second feature signature may be obtained based on second image data of the second slice.

According to an aspect, the method may further comprise
- defining a plurality of first (image) patches in the first slice,
- obtaining for each of the first patches a first feature vector based on the image data of the respective patch,
- defining a plurality of second (image) patches in the second slice, the second patches corresponding to the first patches,
- obtaining, for each of the second patches a second feature vector,
- the step of calculating the similarity signature comprises, for each of the first patches,
- calculating a local similarity based on the first feature vector of the respective first patch and the second feature vector of the corresponding second patch, wherein the local similarity indicates a degree of similarity between the image data of the respective first image patch and the image data of the corresponding second image patch.

According to an aspect, in the step of outputting, a third slice may be generated based on the extracted first slice and/or the extracted second slice, said third slice including an alert for the determined dissimilarity, said alert visualizing the determined dissimilarity between the extracted first slice and the extracted second slice in the third slice.

According to an aspect, in the step of extracting the second slice, the second slice is selected from a plurality of slices comprised in the second medical image based on the first slice and, preferably, based on the image data of the first slice.

By extracting the second slice in consideration of the first slice, a second slice may be determined which "fits" to the first slice, and which is, in other words, readily comparable to the first slice. For instance, such a targeted selection of the second slice may be based on the slice numbers of the first slice in the first medical image and/or second slice in the second medical image. As an alternative, extracting the second slice may be based on an evaluation of the image data and, in particular, on an evaluation of image data similarities again.

According to an aspect, the step of extracting the second slice comprises identifying, from a plurality of slices comprised in the second medical image, the second slice based on degrees of slice similarity between the image data comprised in the first slice and individual slices of the second medical image.

In other words, the concept of a systematic evaluation of image similarities is also used for selecting appropriate first and second slices for the ensuing change quantification. This has the advantage that corresponding slices in first and second medical images may also be determined if the slice numbering is not helpful, e.g., because there is an offset in the underlying image volumes or because the slices in first and second medical images represent image sections of different thicknesses. Thus, a more robust selection of the second slice may be provided.

According to an aspect, the step of identifying the second slice from a plurality of slices of the second medical image comprises:
- extracting an image descriptor from image data of the first slice;
- respectively extracting a corresponding image descriptor from each of a plurality of slices of the second medical image,
- wherein the degrees of slice similarity are respectively based on a comparison between the extracted image descriptors of first and second slices.

The extracted image descriptors may be generated essentially in the same way as explained in connection with the feature vectors. In particular, the image descriptors may also be generated by inputting the slices in a trained machine learning algorithm. However, the image descriptors comprise a different kind of information as compared to the feature vectors. Rather than characterizing individual patches of an image slice, the image descriptors characterize an entire slice.

According to an aspect, in the step of identifying, the one slice of the plurality of slices of the second medical image having the highest degree of slice similarity is identified as the second slice.

With the one slice, a second slice can be identified which is the most similar to the first slice and which, thus, provides a good basis for determining changes in longitudinal examinations of a patient.

According to an aspect, the step of extracting the first slice may comprise selecting the first slice (optionally from a plurality of slices of the first medical image) by the user. This may involve receiving a user input directed to the selection of the first slice from the user via a user interface and extracting the first slice on that basis. Accordingly, the user may select the basis for comparing the first and second medical image by her- or himself and may thus define which parts of the first and second medical image are most important.

Alternatively, the step of extracting the first slice may be carried out automatically. For instance, the selection may be based on an image analysis step directed to identify one or more lesions in the first medical image. This may involve applying in principle known computer aided detection algorithms to the first medical image. Alternatively or additionally, the automated selection may be based on supplementary (non-image) data associated to or comprised in the first medical image. For instance, such supplementary data may comprise one or more previous annotations (optionally pointing to pathological relevant findings) related to the first medical image. As an example, the supplementary data may comprise one or more medical reports and an association may be provided for by a common patient identifier of the first (and second) medical image and the supplementary data. Thus, the automated extraction of the first slice may comprise retrieving the supplementary data (e.g., based on the patient identifier from a database) and extract the first slice on that basis. By automatically extracting the first slice, users may be provided with additional assistance regarding the most relevant parts of potentially huge data sets in medical images.

According to an aspect, the step of determining a dissimilarity between the extracted first slice and the extracted second slice comprises:
generating a first feature vector based on image data comprised in the first slice,
generating a second feature vector based on image data comprised in the second slice,
wherein the step of determining the image similarity comprises calculating a similarity value by evaluating a similarity metric based on the first feature vector and the second feature vector, and
wherein, in the step of determining the dissimilarity, a dissimilarity is determined based on a comparison of the calculated similarity value with a pre-determined threshold.

The feature vector (in other embodiments also referred to as input vector) may be calculated essentially as described before, in particular, by using a trained machine learning algorithm. The similarity metric may be configured to quantify a distance in (feature) vector space between the first and second feature vector. As explained, the dissimilarity identification based on feature vectors allows for a robust identification of changes and is applicable to a comparably huge variety of different medical images without requiring detailed knowledge about the involved organs or underlying rheological properties of the tissue.

According to an aspect, the step of calculating the similarity value is executed for a plurality of different locations in the first slice and in the second slice, wherein the step of determining the dissimilarity comprises identifying a certain location as dissimilar based on a comparison of the calculated similarity value with the pre-determined threshold.

According to an aspect, the plurality of different locations may be related to the plurality of first and second patches as described before, and the similarity values may be the local similarities as introduced before.

According to an aspect, the certain location may be related to one of the image patches described before. Accordingly, the step of calculating may comprise identifying a first image patch in the first slice related to or located at the certain location and identifying a second image patch in the second slice related to or located at the certain location, generating a first feature vector based on image data comprised in the first patch, generating a second feature vector based on image data comprised in the second patch, calculating the similarity value (as local similarity) for the certain location based on the first and second feature vectors, and identifying the certain location as dissimilar based on the similarity value (e.g., by way of a comparison of the similarity value with the pre-determined threshold).

According to an aspect, in the step of outputting, an alert is allocated to a certain location if the certain location is identified as dissimilar.

According to an aspect, the step of determining the image similarity between image data of the first medical image and image data of the second medical image comprises:
applying a trained machine learning algorithm on image data of the first and second medical images, the trained machine learning algorithm is adapted to determine image similarities between images data, and
optionally, wherein the trained machine learning algorithm applies a learned metric to determine image similarities in image data, the trained machine learning algorithm preferably comprising a deep metric learning network.

According to an aspect, the trained machine learning algorithm may be further configured to:
obtain a feature signature from a medical image based on image data of the medical image,
obtain a feature vector of a medical image based on image data of an image patch or a slice of the medical image,
calculate a similarity signature based on two feature signatures, and/or
calculate a similarity value or local similarity based on two feature vectors.

Trained machine learning algorithms, in general, may be seen as mapping input data to output data thereby fulfilling a certain learned task. According to some examples, the machine learning algorithm may be configured to carry out one or more of the following tasks: respectively extract one or more image descriptors, signatures or vectors from first and/or second medical images, compare one or more image descriptors, signatures or vectors, apply a similarity metric, determine one or more degrees of image similarity, similarity signatures, or similarity values (local similarities). The relation between input and output may be governed by one or more (in general: a plethora) of parameters embedded in the trained machine learning algorithm. The values of the parameters may be learned (adapted) during training according to the task, the trained machine learning algorithm will have to fulfill. Other terms for trained machine learning algorithm may be trained mapping specification, mapping specification with trained parameters, function with trained parameters, trained machine learned model, algorithm based on artificial intelligence, trained function, or machine learned algorithm. Applying trained machine learning algorithm may mean inputting the first and second medical images into the trained machine learning algorithm.

According to some examples, the trained machine learning algorithm comprises a machine learned (artificial) neural network, most preferably a convolutional neural network. A neural network is basically built up like a biological neural net, e.g., a human brain. In particular, an artificial neural network comprises an input layer and an output layer. It may further comprise a plurality of layers between input and output layer. Each layer comprises at least one, preferably a plurality of nodes. Each node may be understood as a biological processing unit, e.g., a neuron. In other words, each neuron corresponds to an operation applied to input data. Nodes of one layer may be interconnected by edges or connections to nodes of other layers, in particular, by directed edges or connections. These edges or connections define the data flow between the nodes of the network. In particular, the edges or connections are equipped with a parameter, wherein the parameter is often denoted as "weight". This parameter can regulate the importance of the output of a first node to the input of a second node, wherein the first node and the second node are connected by an edge. In particular, a neural network can be trained. In particular, training of a neural network is performed based on known pairs of input and output values according to a 'supervised learning' technique, wherein the known input values are used as inputs of the neural network, and wherein the corresponding output value of the neural network is compared to the corresponding known output value. The artificial neural network independently learns and adapts the weights for the individual nodes as long as the output values of the last network layer sufficiently correspond to the known output values according to the trainings data. For convolutional neural networks, this technique is also called 'deep learning'. The terms 'neural network' and 'artificial neural network' can be used as synonyms.

A first group of neural network layers may be applied to extract features from the image data comprised in the first and second medical images, in particular, from respective slices and/or patches of first and second medical images. Image data may, for instance, be given in the form of the gray scale and/or color values of each slice/image/patch. The thus extracted features like, contrast, gradients, texture, density, distortion, singularities, patterns, landmarks, masks or the like may form an image descriptor/signature/vector of the respective image/slice/patch. The image descriptors may be fed as input values to a second group of network layers which serve to determine a degree of image similarity/similarity signature/similarity value between two images/slices/patches based on the extracted features. However, both functions of the described neural network may likewise be carried out by separated, individual neural networks. In other words, image analysis for feature extraction can be carried out by a first neural network, and classification according to similarity can be carried out by a second neural network.

In particular, the machine learned neural network may be a convolutional neural network. In particular, the machine learned neural network may be a deep convolutional neural network. According to such implementations, the machine learned neural network comprises one or more convolutional layers and/or one or more deconvolutional layers. Further, the machine learned neural network may comprise one or more pooling layers and/or one or more up-sampling layers. Further, the machine learned neural network may comprise one or more fully connected layers.

The inventors have recognized that, through the use of convolutional layers and/or deconvolutional layers, a neural network can be employed especially efficiently for image processing, since despite many connections between node layers, only a few edge weights (namely the edge weights corresponding to the values of the convolutional kernel) have to be determined by training. With a same number of training data, the accuracy of the neural network can thus also be improved.

According to some examples, the trained machine learning algorithm comprises a distance metric learning network and, in particular, a deep distance metric learning network.

Distance metric learning (or simply, metric learning) aims at automatically constructing task-specific distance or similarity metrics from supervised data, in a machine learning manner. The learned distance metric can then be used to perform various tasks such as, in this case, the identification of similar image data and the quantification of the image similarity. In comparison to the usage of preset distance metrics, learned distance metrics may have the advantage that the learned metric is better adapted to the particular data and task.

Deep (distance) metric learning networks may additionally transform the data into a new feature space with higher discrimination power before a metric (either learned or standard) is applied. The feature space may be such that extracted image features that are semantically similar are mapped onto nearby locations while dissimilar image features are pushed apart using an appropriate distance metric.

According to some examples, the trained machine learning algorithm comprises a Siamese network. According to further examples, the trained machine learning algorithm may comprise a fully convolutional Siamese network.

A Siamese network is a type of neural network that learns to compare two inputs based on a distance or similarity metric such that inputs that are closer in some semantics get a lower distance in comparison to two inputs that are further apart according to the same semantics. The semantics that need to be captured is fed to the network implicitly during the training processes. The semantics can be conceived as the vector space of the image descriptors/signatures/vectors. Accordingly, the semantics determine which image descriptors/signatures/vectors are extracted. The semantics may be extracted using branches of sub-networks with identical structure and parameters. The extracted image descriptors/signatures/vectors may be seen as a representation of the learned semantic. In particular, a Siamese network may comprise a structure with two branches of sub-networks with identical structure and parameters.

Based on a fully convolutional Siamese network at least one convolution processing and at least one pooling processing may be executed on, e.g., a first patch of the first image, thus obtaining image features of that first patch. Further, at least one convolution processing and at least one pooling processing may be executed on, e.g., a second patch of the second image, thus obtaining image features of that second image patch. The output image features after convolution processing and pooling processing may be one or more feature vectors or maps with the same size. Parameters in the convolution processing or the pooling processing, for example, the sizes and number of convolution kernels used for each convolution layer or each pooling layer may be preconfigured via the training process of the fully convolutional Siamese network. In particular, also a fully convolutional Siamese network may comprise a structure with two branches of sub-networks with identical structure and parameters.

According to some examples, the trained machine learning algorithm comprises a triplet network.

Triplet networks may be conceived as an extension of Siamese networks, as triplet networks may comprise three branches of the same feedforward network. When fed with three samples, the network outputs intermediate values in the form of a pair of distances. Thereby, one sample is taken as the reference (or anchor) against which the others are compared. The intermediate values are then fed into a comparator to determine an output. Rather than directly comparing data labels, the triplet network allows learning by comparison of samples which poses lower requirements to the training data and could enable the usage as an unsupervised learning model.

According to some examples, the trained machine learning algorithm is trained using a triplet loss function.

A triplet loss function is a loss function for machine learning algorithms where a reference (anchor) input is compared to a positive (truthy) input and a negative (falsy) input. The distance from the baseline (anchor) input to the positive input is minimized, and the distance from the reference input to the negative input is maximized. Transferred to the present case, the positive input could be an image patch with verified similarity to a given reference image patch. The similarity can, for instance, be verified by a user or by using virtually identical patches. The latter has the advantage that training data can be obtained rather easily. The negative input may for instance be a patch that is less similar to the reference patch than the positive input. In particular, the negative input may be a patch that is not related to the reference patch, e.g., a patch from a different location or patient. By enforcing such an order of similarities, triplet loss models embed (i.e., extract image descriptors) in a way that a pair of similar parches are smaller in distance (or have a higher degree of similarity) than dissimilar patches. One advantage of this approach is that triplet loss functions are very flexible in terms of the training data required.

As an alternative, also other loss functions may be used, such as contrastive loss functions which are computed by contrasting two or more degrees of similarity of patch pairs, or categorical cross-entropy loss functions.

According to an aspect, a computer-implemented method for providing a trained machine learning algorithm is provided. The method comprises a plurality of steps. One step is directed to receiving a trained machine learning algorithm. A further step is directed to providing a first image patch, a second image patch, and a third image patch, wherein first second and third images patches have been extracted from one or more medical images and the second image patch has a greater similarity to the first image patch than the third image patch to the first image patch. A further step is directed to inputting the first, second and third image patches into the trained machine learning algorithm. A further step is directed to determine a first degree of similarity between the first image patch and the second image patch and a second degree of similarity between the first image patch and the third image patch. A further step is directed to adjusting the trained machine learning algorithm such that first degree of similarity is greater than the second degree of similarity.

In other words, the first image patch is used as anchor, while the second image patch is used as positive example and the third image patch is used as negative example. First and second degrees of similarity may, for instance, relate to the distance in feature space between the image descriptors respectively extracted from the three image patches by the trained machine learning algorithm. Adaptations to the trained machine learning algorithm may, for instance, concern what kind of image descriptors are extracted and/or how the extracted image descriptors are compared/processed to derive a degree of similarity. The proposed learning scheme has the advantage that it is less demanding as regards the quality of the training data. Specifically, it does not require absolute labels as to the degrees of similarity which in most cases would be very difficult to provide. Rather, weak labels in the form of a relative indication which one of two patches is more similar to a comparative patch are sufficient to adjust the trained machine learning algorithm. Such weak labels are implicitly comprised in any image study.

According to an aspect, the alert is visualized using a certain color, using augmented reality and/or using virtual reality.

According to an aspect, a computer-implemented method for automated processing of medical images to output alerts for detected dissimilarities in the medical images is proposed. The method comprises:
receiving a first medical image of an anatomical object of a patient, the first medical image being acquired at a first instance of time,
receiving a second medical image of the anatomical object of the patient, the second medical image being acquired at a second instance of time,
determining a dissimilarity between the received first medical image and the received second medical image using a trained machine learning algorithm, the trained machine learning algorithm being adapted to determine two medical images having differences besides posture and motion, in particular besides posture, deformation, motion, variations of anatomy and scanning parameters, as dissimilar and two medical images having no difference or only differences due to posture and motion, in particular besides posture, deformation, motion, variations of anatomy and scanning parameters, as similar, and
outputting a third medical image being generated based on the received first medical image and/or the received second medical image, said third medical image including an alert for each determined dissimilarity, said alert visualizing the determined dissimilarity between the received first medical image and the received second medical image in the third medical image.

By means of the present method, a fast and accurate detection for dissimilarities in medical images is provided. In particular by using said trained machine learning algorithm such as deep neural networks, the present method is unsusceptible for noise and motion. Moreover, only essential changes may be visualized by said alerts to the user, e.g. a radiologist.

According to an aspect, the machine learning algorithm is trained such that two medical images having differences besides posture, deformation, motion, variations of anatomy and scanning parameters are labeled as dissimilar medical images and two medical images having no difference or only differences due to posture, deformation, motion, variations of anatomy and scanning parameters are labeled as similar medical objects.

As the machine learning algorithm is trained regarding the differentiation between similar and dissimilar medical objects, the present method is very unsusceptible regarding noise and motion.

According to an aspect, the step of determining a dissimilarity includes determining at least one dissimilarity between the received first medical image and the received second medical image at a certain location of the anatomical object.

In particular, the present step of determining includes determining a plurality of dissimilarities between the received first medical image and the received second medical image at a plurality of certain locations in the anatomical object. In particular, the plurality of locations may embody a defined matrix in the first and second medical images.

According to an aspect, the method comprises:
extracting a first slice depicting a certain section of the anatomical object from the first medical image,
extracting a second slice depicting the certain section of the anatomical object from the second medical image,
determining a dissimilarity between the extracted first slice and the extracted second slice using the trained machine learning network, the trained machine learning network being adapted to determine two slices having differences besides posture, deformation, motion, variations of anatomy and scanning parameters as dissimilar and two slice having no difference or only differences due to posture, deformation, motion, variations of anatomy and scanning parameters as similar, and
outputting a third slice being generated based on the extracted first slice and/or the extracted second slice, said third slice including an alert for each determined dissimilarity, said alert visualizing the determined dissimilarity between the extracted first slice and the extracted second slice in the further slice.

In particular, the slices as herein descried may be 2D slices and may be extracted at a certain axial plane of the anatomical object In particular, the first medical image may have been captured in the past and the second medical image may be a current medical image for the same patient. As a result, the first slice is a former or older slice and the second slice is a current slice. Moreover, the third slice may be based on the second slice and may additionally include the at least one alert visualizing a determined dissimilarity between the older slice (first slice) and the current slice (second slice). Therefore, any essential change between the two slices, i.e. the older slice and the current slice, is automatically output to the user. In particular by using the present trained machine learning network, also dissimilarities or changes can be visualized to the users which could not be identified by the user manually.

According to an aspect, the machine learning algorithm is trained such that slices having differences besides posture and motion are labeled as dissimilar slices and slices having no difference or only differences due to posture and motion are labeled as similar slices. The present embodiment is unsusceptible to noise and motion, even on a slice level of the medical images taken from the anatomical object.

According to an aspect, the step of determining a dissimilarity includes determining a dissimilarity between the extracted first slice and the extracted second slice at a certain location of the anatomical object.

According to an aspect, the step of determining a dissimilarity between the extracted first slice and the extracted second slice using the trained machine learning network includes:
converting the extracted first slice into a first input vector,
converting the extracted second slice into a second input vector,
inputting the first input vector and the second input vector into the trained machine learning network for determining a distance between the first input vector and the second input vector, and
identifying the first extracted slice and the second extracted slice as similar if the determined distance is smaller than a certain threshold, and identifying the first extracted slice and the second extracted slice as dissimilar if the determined distance is greater than or equal to the certain threshold.

For example, the machine learning algorithm or network may include a plurality of layers. In particular, one layer may be an embedding layer which is configured to execute above-mentioned converting steps, i.e. converting the extracted first slice into a first input vector and converting the extracted second slice into a second input vector. Moreover, said plurality of layers may include a shared LSTM (long shirt term memory) and CNN (convolutional neural network) executing above-mentioned inputting step and above-mentioned identifying step. For example, the extracted first slice is converted into a first input vector h₁ and the extracted second slice is converted into a second input vector h₂. If the two slices are similar, the converted vectors h₁ and h₂ are similar, i.e. a distance ∥h₁ - h₂∥ is small. In other words, locations in the slices are detected where ∥h₁ - h₂∥ is large. Such locations contain changes that are not merely due to posture and motion.

According to an aspect, the step of determining a distance is executed for a plurality of different locations in the first slice and in the second slice, wherein a certain location in the first slice and in the second slice is identified as similar, if the determined distance is smaller than the certain threshold, and the certain location in the first slice and in the second slice is identified as dissimilar, if the determined distance is greater than or equal to the certain threshold.

According to an aspect, the step of determining a distance is executed for a plurality of different locations in the first slice and in the second slice, wherein a certain location in the first slice and the most similar slice among a plurality of the second slices is identified as similar, if the determined distance is smaller than the certain threshold, and the certain location in the first slice and in the second slice is identified as dissimilar, if the determined distance is greater than or equal to the certain threshold.

According to an aspect, an alert is allocated to a certain location if the certain location is identified as dissimilar. Thus, an alert may visualize any location-specific dissimilarity or change besides posture and motion to the user.

According to an aspect, the machine learning network is a neural network, in particular a convolutional neural network (CNN), a Siamese network or a triplet network.

According to an aspect, each of the first medical image, the second medical image and the third medical image is a 3D medical image of the anatomical object of the patient.

According to an aspect, each of the first slice, the second slice and the third slice is a 2D slice.

According to an aspect, the alert is visualized using a certain color, using augmented reality and/or using virtual reality.

According to an aspect, the first medical image and the second medical image are acquired by a medical imaging unit, in particular by a MR scanner or a CT scanner.

According to an aspect, the first instance of time and the second instance of time are different. For example, between the first instance of time and the second instance of time may be months or years.

According to an aspect, a computer-implemented device for automated processing of medical images to output alerts for detected dissimilarities in medical images is proposed. The computer-implemented device comprises:
one or more processing units,
a receiving unit which is configured to receive one or more medical images captured by a medical imaging unit, and
a memory coupled to the one or more processing units, the memory comprising a module configured to perform the method steps of any of the previous aspects optionally using a trained machine learning algorithm.

The respective unit, e.g., the processing unit or the receiving unit, may be implemented in hardware and/or in software. If said unit is implemented in hardware, it may be embodied as a device, e.g., as a computer or as a processor or as a part of a system, e.g., a computer system. If said unit is implemented in software, it may be embodied as a computer program product, as a function, as a routine, as a program code or as an executable object.

The aspects and features according to the described methos are also optional aspects of the device.

According to an aspect, a system for automated processing of medical images to output alerts for detected dissimilarities in medical images is proposed. The system comprising:
one or more servers,
a medical imaging unit coupled to the one or more servers,
the one or more servers comprising instructions, which when executed causes the one or more servers to perform the method steps of the of any of the above aspects.

The aspects related to the described method are also optional aspects of the system.

According to an aspect, a computer program product is proposed, the computer program product comprising machine readable instructions, that when executed by one or more processing units, cause the one or more processing units to perform the method of any of the above aspects.

The aspects and features related to the described method are also optional aspects of the computer program product.

A computer program product, such as a computer program means, may be embodied as a memory card, USB stick, CD-ROM, DVD or as a file which may be downloaded from a server in a network. For example, such a file may be provided by transferring the file comprising the computer program product from a wireless communication network.

According to an aspect, a computer readable medium is proposed on which program code sections of a computer program are saved, the program code sections being loadable into and/or executable in a system to make the system execute the method of the first aspect or of any embodiment of the first aspect when the program code sections are executed in the system.

The aspects and features related to the described method are also optional aspects of the computer readable medium.

The realization by a computer program product and/or a computer-readable medium has the advantage that already existing management systems can be easily adopted by software updates in order to work as proposed by the invention.

Further possible implementations or alternative solutions of the invention also encompass combinations - that are not explicitly mentioned herein - of features described above or below with regard to the embodiments. The person skilled in the art may also add individual or isolated aspects and features to the most basic form of the invention.

Further embodiments, features, and advantages of the present invention will become apparent from the subsequent description and dependent claims, taken in conjunction with the accompanying drawings, in which:
- FIG. 1: illustrates a block diagram of a client-server architecture embodying a system for automated processing of medical images to output alerts for detected dissimilarities in the medical images according to an embodiment of the present invention,
- FIG. 2: illustrates a block diagram of a data processing system embodying a computer-implemented device for automated processing of medical images to output alerts for detected dissimilarities in the medical images,
- FIG. 3: illustrates a flowchart of a first embodiment of a method for automated processing of medical images to output alerts for detected dissimilarities in the medical images,
- FIG. 4: illustrates a flowchart of a second embodiment of a method for automated processing of medical images to output alerts for detected dissimilarities in the medical images,
- FIG. 5: illustrates a flowchart of an embodiment of the step of determining a dissimilarity between an extracted first slice and an extracted second slice,
- FIG. 6: illustrates an example of a generated medical image including an alert visualizing a determined dissimilarity in medical images,
- FIG. 7: illustrates a further example of a generated medical image including an alert visualizing a determined dissimilarity in medical images,
- FIG. 8: illustrates an example of conventional medical images showing a set of corresponding slices of a pair of prior and current chest volumes,
- FIG. 9: illustrates an example of conventional medical images showing a set of corresponding slices of a pair of prior and current brain volumes,
- FIG. 10: illustrates a flowchart of an embodiment of a method for automated processing of medical images to output alerts for detected dissimilarities in the medical images,
- FIG. 11: illustrates a flowchart depicting optional method steps of a method for automated processing of medical images to output alerts for detected dissimilarities in the medical images according to an embodiment,
- FIG. 12: illustrates a data flow diagram depicting data streams in connection with a method for automated processing of medical images to output alerts for detected dissimilarities in the medical images according to an embodiment, and
- FIG. 13: illustrates a flowchart of an embodiment of a method for automated processing of medical images to output alerts for detected dissimilarities in the medical images.

Hereinafter, embodiments for carrying out the present invention are described in detail. The various embodiments are described with reference to the drawings, wherein like reference numerals are used to refer to like elements throughout. In the following description, for purpose of explanation, numerous specific details are set forth in order to provide a thorough understanding of one or more embodiments. It may be evident that such embodiments may be practiced without these specific details.

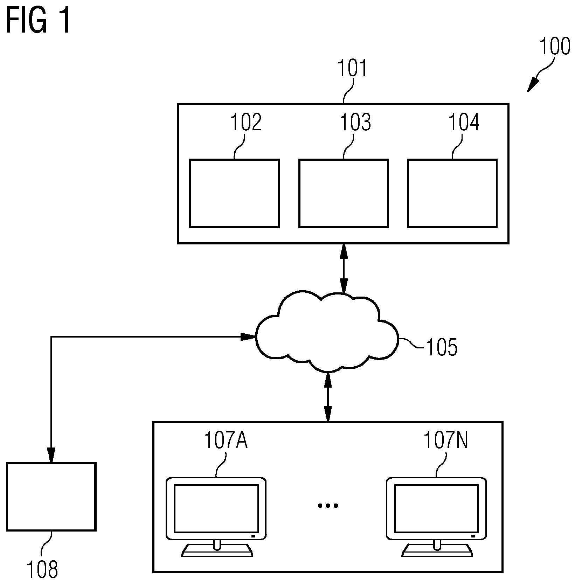

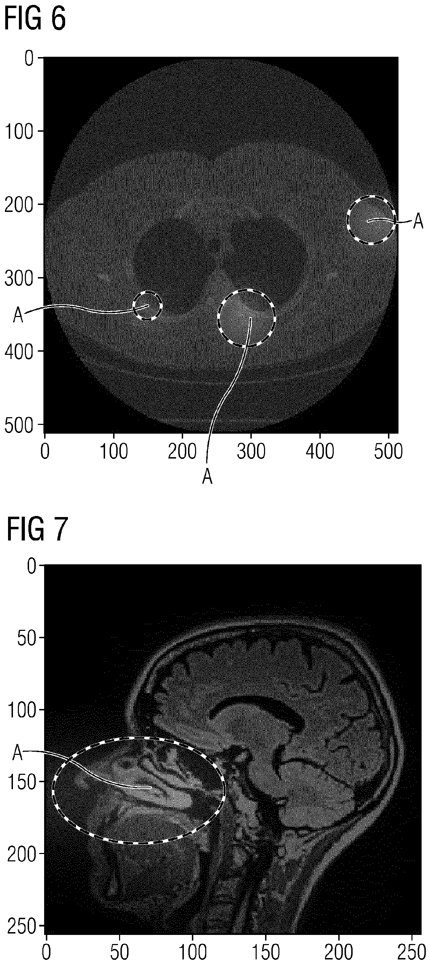

FIG. 1 provides an illustration of a block diagram of a client-server architecture embodying a system for automated processing of medical images to output alerts for detected dissimilarities in the medical images. The client-server architecture 100 comprises a server 101 and a plurality of client devices 107A-N. Each of the client devices 107A-N is connected to the server 101 via a network 105, for example, local area network (LAN), wide area network (WAN), WiFi, etc. In one embodiment, the server 101 is deployed in a cloud computing environment. As used herein, "cloud computing environment" refers to a processing environment comprising configurable computing physical and logical resources, for example, networks, servers, storage, applications, services, etc., and data distributed over the network 105, for example, the internet. The cloud computing environment provides on-demand network access to a shared pool of the configurable computing physical and logical resources. The server 101 may include a medical database 102 that comprises medical images and associated medical data related to a plurality of patients that is maintained by a healthcare service provider. In an embodiment, the medical database 102 comprises images captured by a MR scanner and/or by a CT scanner. The server 101 may include a module 103 that is configured to perform automated processing of medical images to output alerts A (see FIG. 6 and FIG. 7) for detected dissimilarities in medical images. Additionally, the server 101 may include a network interface 104 for communicating with the client device 107A-N via the network 105.

The client devices 107A-N are user devices, used by users, for example, medical personnel such as a radiologist, pathologist, physician, etc. In an embodiment, the user device 107A-N may be used by the user to receive medical images associated with the patient. The data can be accessed by the user via a graphical user interface of an end user web application on the user device 107A-N. In another embodiment, a request may be sent to the server 101 to access the medical images associated with the patient via the network 105. An imaging unit 108 may be connected to the server 101 through the network 105. The unit 108 may be a medical imaging unit 108 capable of acquiring a plurality of medical images. The medical imaging unit 108 may be, for example, a scanner unit such as a magnetic resonance imaging unit, computed tomography imaging unit, an X-ray fluoroscopy imaging unit, an ultrasound imaging unit, etc.

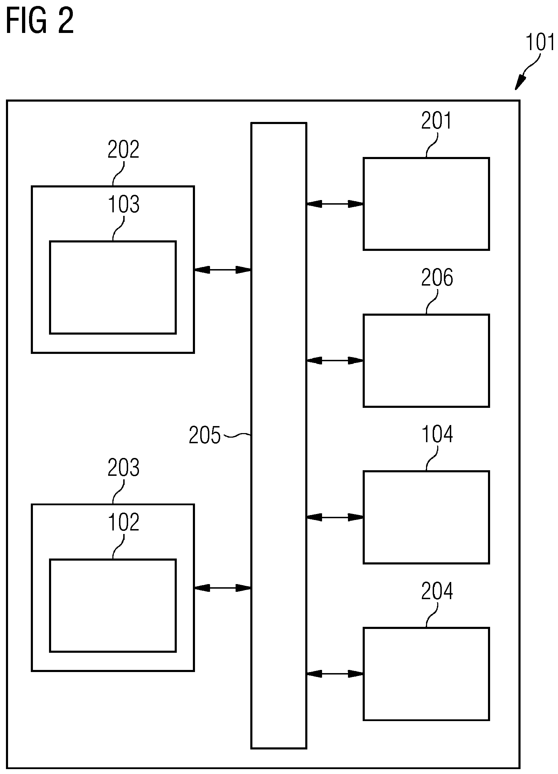

FIG. 2 is a block diagram of a data processing system 101 in which an embodiment can be implemented, for example, as a system 101 for automated processing of medical images to output alerts A for detected dissimilarities in the medical images, configured to perform the processes as described therein. It is appreciated that the server 101 is an exemplary implementation of the system in FIG. 2. In FIG. 2, said data processing system 101 comprises a processing unit 201, a memory 202, a storage unit 203, an input unit 204, an output unit 206, a bus 205, and a network interface 104.

The processing unit 201, as used herein, means any type of computational circuit, such as, but not limited to, a microprocessor, microcontroller, complex instruction set computing microprocessor, reduced instruction set computing microprocessor, very long instruction word microprocessor, explicitly parallel instruction computing microprocessor, graphics processor, digital signal processor, or any other type of processing circuit. The processing unit 101 may also include embedded controllers, such as generic or programmable logic devices or arrays, application specific integrated circuits, single-chip computers, and the like.

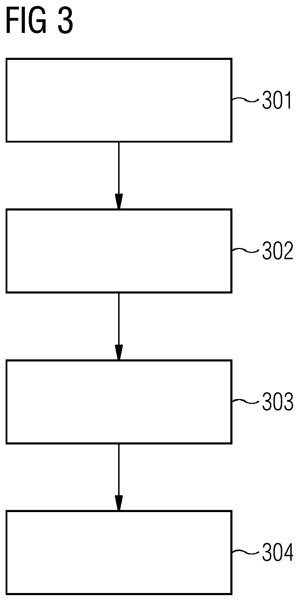

The memory 202 may be volatile memory and non-volatile memory. The memory 202 may be coupled for communication with said processing unit 201. The processing unit 201 may execute instructions and/or code stored in the memory 202. A variety of computer-readable storage media may be stored in and accessed from said memory 202. The memory 202 may include any suitable elements for storing data and machine-readable instructions, such as read only memory, random access memory, erasable programmable read only memory, electrically erasable programmable read only memory, a hard drive, a removable media drive for handling compact disks, digital video disks, diskettes, magnetic tape cartridges, memory cards, and the like. In the present embodiment, the memory 201 comprises a module 103 stored in the form of machine-readable instructions on any of said above-mentioned storage media and may be in communication to and executed by processing unit 201. When executed by the processing unit 201, the module 103 causes the processing unit 201 to automatically process medical images for outputting alerts A for detected dissimilarities in the medical images. Method steps executed by the processing unit 201 to achieve the abovementioned functionality are elaborated upon in detail in FIG. 3, 4, and 5.

The storage unit 203 may be a non-transitory storage medium which stores a medical database 102. The medical database 102 is a repository of medical images and associated medical data sets related to one or more patients that is maintained by a healthcare service provider. The input unit 204 may include input means such as keypad, touch-sensitive display, camera (such as a camera receiving gesture-based inputs), etc. capable of receiving input signal such as a medical image. The bus 205 acts as interconnect between the processor 201, the memory 202, the storage unit 203, the input unit 204, the output unit 206 and the network interface 104.

Those of ordinary skilled in the art will appreciate that said hardware depicted in FIG. 1 may vary for particular implementations. For example, other peripheral devices such as an optical disk drive and the like, Local Area Network (LAN)/ Wide Area Network (WAN)/ Wireless (e.g., Wi-Fi) adapter, graphics adapter, disk controller, input/output (I/O) adapter also may be used in addition or in place of the hardware depicted. Said depicted example is provided for the purpose of explanation only and is not meant to imply architectural limitations with respect to the present disclosure.

A data processing system 101 in accordance with an embodiment of the present disclosure may comprise an operating system employing a graphical user interface. Said operating system permits multiple display windows to be presented in the graphical user interface simultaneously with each display window providing an interface to a different application or to a different instance of the same application. A cursor in said graphical user interface may be manipulated by a user through a pointing device. The position of the cursor may be changed and/or an event such as clicking a mouse button, generated to actuate a desired response.

One of various commercial operating systems, such as a version of Microsoft Windows^{™}, a product of Microsoft Corporation located in Redmond, Washington may be employed if suitably modified. Said operating system is modified or created in accordance with the present disclosure as described. Disclosed embodiments provide systems and methods for processing medical images.

FIG. 3 illustrates a flowchart of a first embodiment of a method for automated processing of medical images to output alerts A (see FIG. 6 and FIG. 7, for example) for detected dissimilarities in the medical images. In particular, the method of FIG. 3 may be executed by above discussed module 103. The embodiment of FIG. 3 includes the method steps 301 - 304:
In step 301, a first medical image of an anatomical object of a patient is received, the first medical image being acquired by a medical imaging unit 108 at a first instance of time.
In step 302, a second medical image of the same anatomical object of the patient is received, the second medical image being acquired at a second instance of time. The first instance of time and the second instance of time are different. For example, the first medical image was captured in September 2020, and the second medical image was captured in September 2021. In particular, the first medical image and the second medical image are acquired by the medical imaging unit 108, in particular by an MR scanner or a CT scanner.
In step 303, a dissimilarity between the received first medical image and the received second medical is determined using a trained machine learning network. Determining a dissimilarity may include measuring or calculating the similarity between the two medical images. The machine learning network may be a neural network, in particular a convolutional neural network (CNN), a Siamese network or a triplet network. The trained machine learning network is adapted to determine two medical images having differences besides posture and motion as dissimilar and two medical images having no difference or only differences due to posture deformation, motion, variations of anatomy and scanning parameters as similar.

In the training phase, the machine learning network is trained such two medical images having differences besides posture and motion are labeled as dissimilar medical images and two medical images having no difference or only differences due to posture deformation, motion, variations of anatomy and scanning parameters are labeled as similar medical images.

In step 303, determining a dissimilarity may include determining at least one dissimilarity between the received first medical image and the received second medical image at a certain location of the anatomical object. In particular, for a plurality of corresponding locations in the first medical image and the second medical image, a similarity is measured or calculated for the corresponding locations of a pair of a prior medical image (first medical image) and a current medical image (second medical image).

In step 304, a third medical image being generated based on the received first medical image and/or received second medical image is output to a user, for example by one of the client devices 107A - 107N as shown in FIG. 1. The output third medical image may include an alert A for each determined dissimilarity. For example, the medical image of FIG. 6 corresponding to a third medical image includes three alerts A visualizing three different dissimilarities. For example, the output third medical image may be based on the current medical image (second medical image) including the generated alerts A, three colored sections (illustrated by circles in the example of FIG. 6).

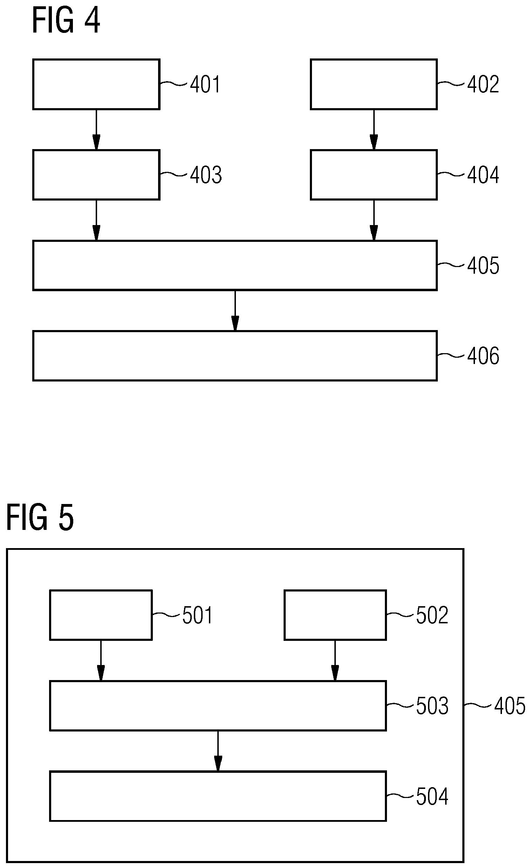

FIG. 4 illustrates a flowchart of a second embodiment of a method for automated processing of medical images to output alerts A for detected dissimilarities in the medical images. Also, the method of FIG. 4 may be executed by above discussed module 103.

The method of FIG. 4 comprises the following method steps 401 - 406:
In step 401, a first medical image of an anatomical object of a patient is received, the first medical image being acquired by a medical imaging unit 108 at a first instance of time.
In step 402, a second medical image of the same anatomical object of the same patient is received, the second medical image being acquired by the medical imaging unit 108 at a second instance of time. The first instance of time and the second instance of time are different to each other.
In step 403, a first slice depicting a certain section of the anatomical object is extracted from the first medical image.

In an analogous way, in step 404, a second slice corresponding to said first slice and depicting the same certain section of the same anatomical object is extracted from the second medical image. As a result, the first slice and the second slice are showing the same object or part of the same object, with changes in time as they are captured at different instances of time.

In step 405, a dissimilarity between the extracted first slice and the extracted second slice is determined using the trained machine learning network, the trained machine learning network being adapted to determine two slices having differences besides posture deformation, motion, variations of anatomy and scanning parameters as dissimilar and two slices having no difference or only differences due to posture in motion as similar.

In this regard, FIG. 5 shows an embodiment of the determining step 405 of FIG. 4. According to FIG. 5, determining a dissimilarity between the extracted first slice and the extracted second slice using the trained machine learning network includes the following method steps 501 - 504:
In step 501, the extracted first slice is converted into a first input vector configured for the trained machine learning network.
In step 502, the extracted second slice is converted into a second input vector configured for the trained machine learning network.
In step 503, the first input vector and the second input vector are input into the trained machine learning network for determining a distance d between the first input vector and the second input vector.
In step 504, the first extracted slice and the second extracted slice are identified as similar, if the determined distance d is smaller than a certain threshold. In contrast, if the determined distance is greater than or equal to the certain threshold, the first extracted slice and the second extracted slice are identified as dissimilar.

For example, each of the two slices is converted to a respective input vector h₁ and h₂ by the neural network being an example for the trained machine learning network. The neural network may be implemented as a Siamese network or a triplet network. If the two slices are similar, the converted vectors h₁ and h₂ are similar, i.e., the distance ∥h₁ - h₂∥ is small. In particular, if locations in the slices are detected where ∥h₁ - h₂∥ is large, such locations contain changes that are not merely due to posture and motion. In the training phase, a label Y may be set 1, if the slices are annotated as similar, and Y = 0, if they are dissimilar.

Coming back to FIG. 4 and the following last step 406. In step 406, a third slice being generated based on the extracted first slice and/or the extracted second slice is output, for example by a client device 107A - 107N as shown in FIG. 1. The third slice may be based on the current slice (second slice) and may include an alert A for each determined dissimilarity, said alert A visualizing the determined dissimilarity between the extracted first slice and the extracted second slice in the third slice.

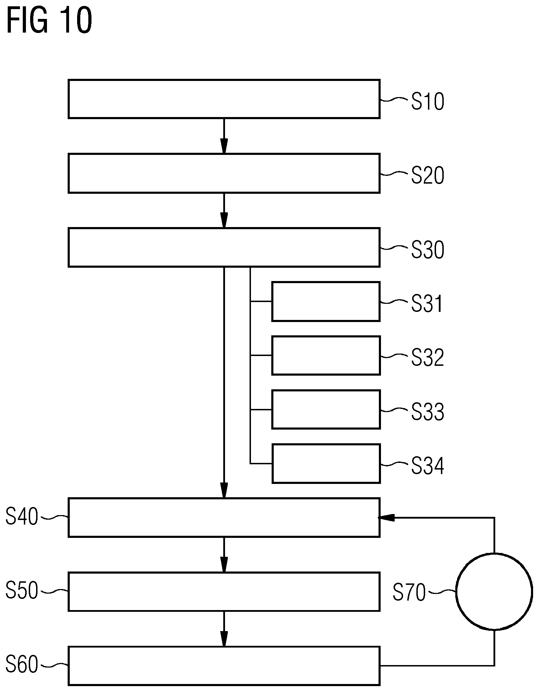

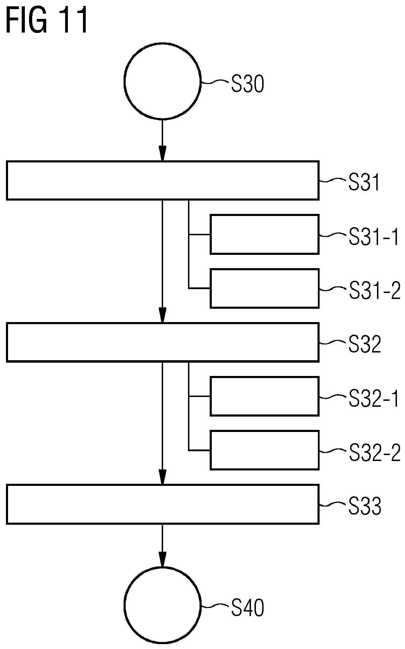

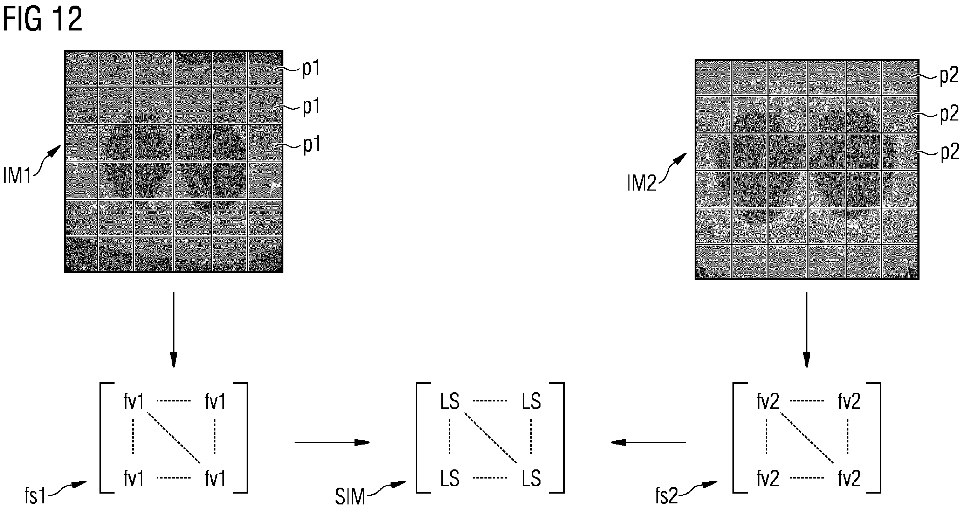

FIG 10 depicts a method for automated processing of medical images IM1, IM2 to output alerts A according to a further embodiment. Additional optional method steps are depicted in FIG. 11. Corresponding data streams are illustrated in FIG. 12. The method comprises several steps. The order of the steps does not necessarily correspond to the numbering of the steps but may also vary between different embodiments of the present invention.

At step S10, the first medical image IM1 is received. In the embodiment of FIG. 10, the image data comprised in the first medical image IM1 relates to a two-dimensional image of a body part of a patient. The two-dimensional image may have been acquired using one of the imaging modalities mentioned above. In particular, the two-dimensional image may be an image which has been extracted from a previous image study of the patient. Further, the two-dimensional image IM1 may be a so-called key-image which has been generated from a previous study and/or has been included in a corresponding medical report. Step S10 may involve manually selecting the patient case by the user with a user interface and/or retrieving the first medical image IM1 from a medical information system. Further, step S10 may involve automatically selecting the first medical image IM1 based on the case and the task at hand.

At step S20, the second medical image IM2 is received. This may involve selecting the second medical image IM2 from a plurality of available data sets of a patient which may be stored in a medical information system. The selection may be performed manually by a user, e.g., by selecting appropriate images IM2 in a user interface. Moreover, the selection may be carried out automatically or semiautomatically by the system for users which need more assistance. In particular, this may involve automatically querying connected databases for appropriate images, in particular, based on the first medical image IM1. For instance, a user may select the first medical image IM1 and step S120 may then comprise automatically retrieving an appropriate second medical image IM2 which could be useful for reading the first medical image IM1. To this end, a data identifier, such as a patient or case ID, may be extracted from the first medical image IM1 and used to query a medical information system for appropriate associated information.

Step S30 is concerned about quantifying an image similarity between the first medical image IM1 and the second medical IM2. In particular, this may involve quantifying image similarities in a spatially resolved manner, i.e., for a plurality of different locations of the first and second medical image IM1, IM2.

In the following, steps S31 to S35 set out some exemplary embodiments, how the determination of image similarities of step S30 may be put into practice. They provide several examples in this regard but are not to be considered as limiting the scope of step S30 as they do not exclude additional possibilities how a similarity between image data can be determined. For instance, as an alternative to an explicit extraction of image descriptors of first and second images IM1, IM2 and their ensuing comparison, a Fourier-based analysis scheme may be implemented. Here, individual images or image regions would be transformed into the Fourier space and a similarity would be calculated by applying mathematical convolution functions. Moreover, an additional image registration step may be performed in order to align the first and the second medical image before determining image similarities in step S30.

An optional step S34 is directed to providing a trained machine-learning algorithm which is configured to determine and, in particular, quantify an image similarity between image data of medical images. In particular, the trained machine learning algorithm may be configured to carry out any one of steps S31-S33 (including further optional sub-steps). It is to be understood, however, that the steps S31-S33 may also be carried out without the use of trained functions, i.e., by image analysis algorithms with hard-coded functionalities which implement one or more deterministic rules, e.g., for selecting features from image data and determining degrees of similarity on that basis.

At step S31, a first feature signature fs1 is generated from the first medical image IM1. The first feature signature fs1 may comprise the representative or characterizing features of the first image IM1 in the form of one or more feature vectors fv1. Such image features may be generated by image analysis methods comprising the identification, analysis and/or measurement of objects, local and or global structures and/or textures present in any image data comprised in the first medical image IM1. The generated image feature signature fs1 may comprise an anatomical feature and/or structure, like e.g. the presence of a landmark or the size of an organ or a structure, texture and/or density of an identified tissue or organ and so forth. The image feature signature fs1 may likewise comprise a parameter characterizing a color and/or grey scale scheme or contrast characteristics or local gray scale gradients present in the analyzed image data. The image feature signature fs1 preferably comprise not only one but numerous features which as a sum characterize the analyzed image.

To allow for the systematic local characterization of the first image IM1, the feature extraction of step S31 may be carried out at a plurality of distinct locations. To this end, a plurality of first image patches p1 may be defined in the first medical image at optional step S31-1. That followed, in optional step S31-2, first feature vectors fv1 may be calculated for each of the first image patches p1. The plurality of the first feature vectors fv1 thus generated may be included in the fist feature signature fs1.

The first image patches p1 may be rectangular or quadratic. The actual definition of the image patches P may comprise partitioning the first medical image IM1 by applying a grid, wherein the grid size defines the patch size. Alternatively, the first image patches p1 may be defined using a sliding window algorithm with or without overlapping windows. In step S31-1, an array of two-dimensional image patches p1 may be obtained, which image patches p1 are distributed across the first medical image IM1. Accordingly, the ensuing processing of step S31-2 will also produce an array of first feature vectors fv1 comprising a feature vector fv1 for each first image patch p1. The array of first feature vectors fv1 may have the same format as the array of first image patches p1 obtained at the end of step S31-1.

At step S32, a second feature signature fs2 corresponding to the first feature signature fs1 is extracted from the second medical image IM2. Thereby, the generation of the second feature signature fs2 may follow the same course of action as described in connection with the generation of the first feature signature fs1 at step S31. Accordingly, step S32 may comprise an optional step S32-1 of defining a plurality of second image patches p2 in the second medical image IM2 and an optional step S32-2 of calculating a second feature vector fv2 for each second image patch p2. Both of these steps S32-1 and S32-2 may be implemented in essentially the same way as described in connection with steps S31-1 and S31-2. In particular, the second image patches p2 may be defined so as to correspond to the first image patches p1. In particular, the second image patches p2 are preferably located at the same locations as the first image patches p1 and have preferably the same sizes as the first image patches p1.

At step S33, the first feature signature fs1 is compared to the second feature signature fs2 in order to determine the image similarity between the first IM1 and the second medical image IM2. According to some examples, the comparison of steps S33 may comprise calculating a similarity or distance metric representing a similarity between the first feature signature fs1 and the second feature signature fs2. The result of the calculation may be provided in the form of a similarity signature SIM comprising a plurality of individual similarity values LS.

In particular, step S33 may comprise comparing individual first fv1 and second feature vectors fv2 of (spatially) corresponding first p1 and second image patches p2. Specifically, the comparison of individual feature vectors fv1, fv2 may comprise calculating a similarity or distance metric representing a similarity between the respective first and second feature vectors fv1, fv2. This may result in a local similarity value (or short: local similarity) LS for each pair of first and second image patches p1, p2. Thus, the similarity signature SIM may be conceived as an array of local similarities LS which has the same format as the arrays for the first and second patches p 1, p2 and the arrays of first and second feature vectors fv1, fv2.

In some examples, the similarity metric may be a distance in vector space between individual feature vectors fv1, fv2. For example, the distance may be the Euclidean distance between the two points in vector space that the respective feature vectors fv1, fv2 represent. In some other examples, the similarity metric may be based on the L1 norm of the respective feature vectors fv1, fv2. In some further examples, other similarity metrics may be used, such as a cosine similarity between the feature vectors fv1, fv2. For each patch p2 of the second medical image IM2, the similarity metric may represent how similar the patch p2 is to the corresponding patch p1 of the first image IM1. In other words, the similarity metric expresses (quantifies) a degree of similarity between individual image patches.

The image similarities determined in step S30 may be used to detect those portions of first and second images IM1, IM2 that are dissimilar and therefore indicate pathological changes which cannot be explained by normal tissue deformations which would still be found to be comparably similar.

This idea is exploited in step S40 in which a dissimilarity is determined based on the calculated image similarity between the first and second medical image IM1, IM2. Thereby the similarity signature SIM or individual similarity values LS therein comprised may be compared to a pre-determined threshold. Specifically, each local similarity value LS may be compared to the pre-determined threshold giving a spatially resolved readout potentially existing dissimilarities. Pre-determined may mean that one and the same threshold is used for each local similarity LS. The threshold may be an empirically determined threshold. According to other examples, the threshold may be a learned threshold provided by the trained machine-learning algorithm. According to still other examples, the threshold may be interactively set by the user via a corresponding user input received via a user interface. With that, the user may dynamically adapt the sensitivity of the dissimilarity detection (c.f., step S60).

At step S50, the dissimilarities found in step S40 are outputted. The output may be essentially implemented as described before in connection with steps 304 and 406. As explained, the output may be in the form of a visualization comprising one or more visual alerts A indicating the found dissimilarities in a rending based on image data of the first and/or second medical image IM1, IM2.

At optional step S60, a user input is received which is directed to set the pre-determined threshold and the threshold is set based on the user input. That followed in optional step S70, the new threshold may be fed back to step S40 to recalibrate the detection of dissimilarities - which may then be the basis for adapting the output of step S50.

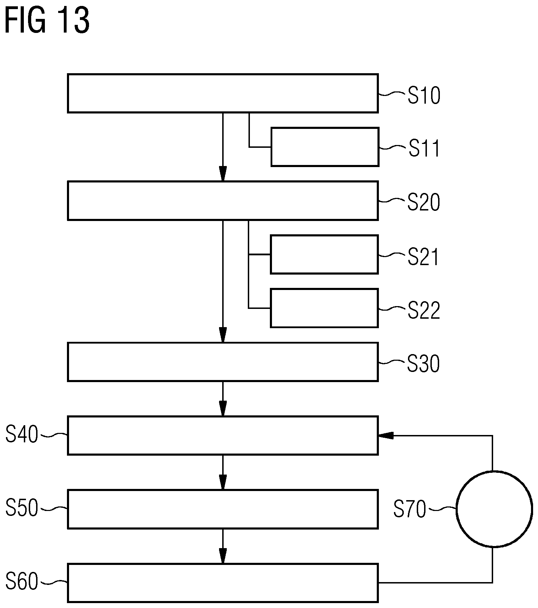

FIG 13 depicts a method for automated processing of medical images IM1, IM2 to output alerts A according to a further embodiment. The method comprises several steps. The order of the steps does not necessarily correspond to the numbering of the steps but may also vary between different embodiments of the present invention. Methods steps indicated with like reference numerals as in FIG 10 correspond to the method steps introduced and explained in connection with the embodiment of FIG 10 (including any optional steps). Further, individual steps or a sequence of steps may be repeated.

The embodiment of FIG 13 is based on the assumption that the first and the second medical image IM1, IM2 are 3D medical image data sets which respectively depict an image volume.

As in the embodiment of FIG 10, the first medical image is received at step S10. However, in the embodiment of FIG 13, step S10 includes an additional sub-step S11 of extracting form the first medical image a first slice. For instance, the first slice may be extracted based on a user input directed to identify a certain section of image volume depicted in the first medical image IM1. Alternatively, the first slice may be extracted automatically, e.g., based on a presumed relevance of the first slice for the follow-up reading. For instance, already existing annotations related to a certain slice of the first image IM1 may be exploited in this regard.

At step S20, the second medical image IM2 is received as described before. That followed, in an optional sub-step S22, the second medical image IM2 may be resampled based on the first medical image IM1 for bringing it into a better shape for the ensuing comparison of the image contents of the two data sets. This may involve defining a plurality of slices of appropriate slice thickness and stacking direction in the second medical image IM2. This may further involve resampling already existing slices in the second medical image IM such that they have an appropriate slice thickness and orientation. Moreover, step S22 may comprise other image processing steps for improving the comparability of the second medical image IM2 with the first medical image IM1. This may comprise reading the image processing steps done to the image data comprised in the first medical image IM1 (which may be encoded in a meta-data file of the first medical image IM1, for instance) and applying the same image processing steps to the second medical image IM2.

At sub-step S21 of step S20, a second slice is extracted from the second image IM2 based on the first slice. An idea of this step S21 is to select the second slice such that it can be readily compared to the first slice. Accordingly, the second slice should depict (roughly) the same body region of the patient as the first slice. This task may, again, be solved by identifying image information in the second medical image IM2 which corresponds to the first slice or, in other words, has a high degree of image similarity with the first slice.

To this end, an image descriptor may be generated from the first slice. The image descriptor may comprise the representative or characterizing features of the first slice in the form of a feature vector essentially as described before in connection with FIG. 10. In contrast to the feature vectors fv1, fv2 which are based on individual patches, the feature vector of an entire slice provides a much coarser readout. Thus, even if individual slices differ by local dissimilarities, they may still be recognized as similar based on encoding their contents in a slice-based feature vector. Subsequently, corresponding image descriptors are extracted from a plurality of candidate slices of the second medical image IM2. The image descriptors of the second medical image IM2 may be generated in the same way as the image descriptor for the first slice. That followed, the image descriptor extracted from the first slice is compared to the image descriptors D-2 extracted from the second medical IM2. According to some examples, this comparison may comprise determining a similarity or distance metric representing a slice similarity between the image descriptor of the first slice and a respective one of the image descriptors extracted from the second medical image IM2 essentially as described before in connection with FIG. 10. Here, the similarity metric expresses (quantifies) a degree of slice similarity between the first slice and a respective slice of the second medical image IM2. The similarities determined may be used to select the slice of the second medical image IM2 having the greatest similarity to the image data in the first slice. This slice may then be extracted as second slice in step S21.

The ensuing steps S30-S70 correspond to the steps introduced in connection with FIG. 10, wherein the step S30 is based on image data of the first slice and the second slice. Specifically, for determining a similarity or dissimilarity of a certain location in the first slice and the second slice, the patches may be defined on the basis of which a spatially resolved feature vector extraction and comparison may be implemented.

The following points are also part of the disclosure:
1. A computer-implemented method for automated processing of medical images to output alerts (A) for detected dissimilarities in the medical images, the method comprising:
   receiving (301, 401) a first medical image of an anatomical object of a patient, the first medical image being acquired at a first instance of time,
   receiving (302, 402) a second medical image of the anatomical object of the patient, the second medical image being acquired at a second instance of time,
   determining (303, 405) a dissimilarity between the received first medical image and the received second medical image using a trained machine learning algorithm, the trained machine learning network being adapted to determine two medical images having differences besides posture, deformation, motion, variations of anatomy and scanning parameters as dissimilar and two medical images having no difference or only differences due to posture, deformation, motion, variations of anatomy and scanning parameters as similar, and
   outputting (304, 406) a third medical image being generated based on the received first medical image and/or the received second medical image, said third medical image including an alert (A) for each determined dissimilarity, said alert (A) visualizing the determined dissimilarity between the received first medical image and the received second medical image in the third medical image.
2. The computer-implemented method according to 1,
   wherein the machine learning algorithm is trained such that two medical images having differences besides posture and motion are labeled as dissimilar medical images and two medical images having no difference or only differences due to posture, deformation, motion, variations of anatomy and scanning parameters are labeled as similar medical objects.
3. The computer-implemented method according to 1 or 2,
   wherein the step of determining (303) a dissimilarity includes determining at least one dissimilarity between the received first medical image and the received second medical image at a certain location of the anatomical object.
4. The computer-implemented method of one of points 1 to 3, comprising
   extracting (403) a first slice depicting a certain section of the anatomical object from the first medical image,
   extracting (404) a second slice depicting the certain section of the anatomical object from the second medical image,
   determining (405) a dissimilarity between the extracted first slice and the extracted second slice using the trained machine learning network, the trained machine learning network being adapted to determine two slices having differences besides posture, deformation, motion, variations of anatomy and scanning parameters as dissimilar and two slices having no difference or only differences due to posture, deformation, motion, variations of anatomy and scanning parameters as similar, and
   outputting (406) a third slice being generated based on the extracted first slice and/or the extracted second slice, said third slice including an alert for each determined dissimilarity, said alert (A) visualizing the determined dissimilarity between the extracted first slice and the extracted second slice in the further slice.
5. The computer-implemented method according to 4,
   wherein the machine learning algorithm is trained such that slices having differences besides posture and motion are labeled as dissimilar slices and slices having no difference or only differences due to posture, deformation, motion, variations of anatomy and scanning parameters are labeled as similar slices.
6. The computer-implemented method according to 4 or 5,
   wherein the step of determining (405) a dissimilarity includes determining a dissimilarity between the extracted first slice and the extracted second slice at a certain location of the anatomical object.
7. The computer-implemented method according to 5 or 6,
   wherein the step of determining (405) a dissimilarity between the extracted first slice and the extracted second slice using the trained machine learning algorithm includes:
   converting (501) the extracted first slice into a first input vector,
   converting (502) the extracted second slice into a second input vector,
   inputting (503) the first input vector and the second input vector into the trained machine learning network for determining a distance between the first input vector and the second input vector, and
   identifying (504) the first extracted slice and the second extracted slice as similar if the determined distance is smaller than a certain threshold, and identifying the first extracted slice and the second extracted slice as dissimilar if the determined distance is greater than or equal to the certain threshold.
8. The computer-implemented method according to 7,
   wherein the step (503) of determining a distance is executed for a plurality of different locations in the first slice and in the second slice,
   wherein a certain location in the first slice and in the second slice is identified as similar, if the determined distance is smaller than the certain threshold, and the certain location in the first slice and in the second slice is identified as dissimilar, if the determined distance is greater than or equal to the certain threshold.
9. The computer-implemented method according to 8,
   wherein an alert (A) is allocated to a certain location if the certain location is identified as dissimilar.
10. The computer-implemented method of one of points 1 to 9,
   wherein the machine learning algorithm is a neural network, in particular a convolutional neural network (CNN), a Siamese network or a triplet network.
11. The computer-implemented method of one of points 1 to 10,
   wherein the alert (A) is visualized using a certain color, using augmented reality and/or using virtual reality.
12. A computer-implemented device (101) for automated processing of medical images to output alerts (A) for detected dissimilarities in medical images, the computer-implemented device (101) comprising:
   one or more processing units (201),
   a receiving unit (204) which is configured to receive one or more medical images captured by a medical imaging unit (108), and
   a memory (202) coupled to the one or more processing units (201), the memory (202) comprising a module (103) configured to perform the method steps as claimed in any one of points 1 to 11 using a trained machine learning network.
13. A system (100) for automated processing of medical images to output alerts (A) for detected dissimilarities in medical images, the system (100) comprising:
   one or more servers (101),
   a medical imaging unit (108) coupled to the one or more servers (101),
   the one or more servers (101) comprising instructions, which when executed causes the one or more servers (101) to perform the method steps as claimed in any one of points 1 to 11 using a trained machine learning network.
14. A computer program product comprising machine readable instructions, that when executed by one or more processing units (201), cause the one or more processing units (201) to perform method steps according to any one of points the 1 to 11.
15. A computer readable medium on which program code sections of a computer program are saved, the program code sections being loadable into and/or executable in a system (100) to make the system (100) execute the method steps according to any one of the points 1 to 11 when the program code sections are executed in the system (100).

The foregoing examples have been provided merely for the purpose of explanation and are in no way to be construed as limiting of the present invention disclosed herein. While the invention has been described with reference to various embodiments, it is understood that the words, which have been used herein, are words of description and illustration, rather than words of limitation. Further, although the invention has been described herein with reference to particular means, materials, and embodiments, the invention is not intended to be limited to the particulars disclosed herein, rather, the invention extends to all functionally equivalent structures, methods and uses, such as are within the scope of the appended claims. Those skilled in the art, having the benefit of the teachings of this specification, may effect numerous modifications thereto and changes may be made without departing from the scope and spirit of the invention in its aspects.

## Claims

1. A computer-implemented method for automated processing of medical images to output alerts (A) for detected dissimilarities in the medical images, the method comprising:
receiving (301, 401, S10) a first medical image (IM1) of an anatomical object of a patient, the first medical image (IM1) being acquired at a first instance of time,
receiving (302, 402, S20) a second medical image (IM2) of the anatomical object of the patient, the second medical image (IM2) being acquired at a second instance of time,
determining (S30) an image similarity between image data of the first medical image (IM1) and image data of the second medical image (IM2),
determining (303, 405, S40) a dissimilarity between the first medical image (IM1) and the second medical image (IM2) based on the image similarity, and
outputting (S50) an alert (A) for the dissimilarity.

2. Method according to claim 1, wherein
- the step of outputting (S50) comprises outputting (304, 406) a third medical image being generated based on the received first medical image (IM1) and/or the received second medical image (IM2), said third medical image including an alert (A) for each determined dissimilarity, said alert (A) visualizing the determined dissimilarity between the received first medical image and the received second medical image in the third medical image.

3. Method according to any one of the preceding claims, wherein
the step of determining (S40) the dissimilarity comprises,
comparing the image similarity with a pre-determined threshold.

4. Method according to any one of the preceding claims, wherein
- the step of determining (S30) the image similarity comprises:
- obtaining (S31) a first feature signature (fs1) from the first medical image (IM1) based on first image data of the first medical image (IM1),
- obtaining (S32) a second feature signature (fs2) from the second medical image (IM2) based on second image data of the second medical image (IM2), and
- calculating (S33) a similarity signature (SIM) based on the first feature signature (fs1) and the second feature signature (fs2), the similarity signature (SIM) indicating the image similarity between first and second image data.

5. Method according to claim 4, wherein
- the step of determining (S31) the first feature signature (fs1) comprises:
- defining (S31-1) a plurality of first patches (p1) in the first medical image (IM1),
- obtaining (S31-2) for each of the first patches (p1) a first feature vector (fv1) based on the image data of the respective patch (p1), wherein the first feature signature (fs1) includes the plurality of first feature vectors (fv1),
- the step of determining (S40) the second feature signature (fs2) comprises:
- defining (S32-1) a plurality of second patches (p2) in the second medical image (IM2), the second patches (p2) corresponding to the first patches (p1),
- obtaining (S32-2), for each of the second patches (p2) a second feature vector (fs2), wherein the second feature signature (fs2) includes the plurality of second feature vectors (fv2),
- the step of calculating (S33) the similarity signature (SIM) comprises, for each of the first patches (p1),
- calculating a local similarity (LS) based on the first feature vector (fv1) of the respective first patch (p1) and the second feature vector (fv2) of the corresponding second patch (p2), wherein the local similarity (LS) indicates a degree of similarity between the image data of the respective first image patch (p1) and the image data of the corresponding second image patch (p2).

6. Method according to claim 5, wherein
the step of determining (S40) the dissimilarity comprises identifying dissimilar pairs of first and second image patches (p1, p2) based on the local similarities (LS).

7. Method according to any one of the preceding claims, comprising
extracting (403, S11) a first slice depicting a certain section of the anatomical object from the first medical image (IM1),
extracting (404, S21) a second slice depicting the certain section of the anatomical object from the second medical image (IM2),
wherein the image similarity is an image similarity between image data of the first slice and image data of the second slice.

8. Method according to claim 7 in combination with any one of the claims 5 or 6, wherein
- in the step of obtaining (S31) the first feature signature (fs1) from the first medical image (IM1), the first feature signature (fs1) is obtained based on first image data of the first slice,
- in the step of obtaining (S32) the second feature signature (fs2) from the second medical image (IM2), second feature signature (fs2) is obtained based on second image data of the second slice.

9. Method according to any one of claims 7 or 8, wherein
- the step of extracting (S21) the second slice comprises identifying, from a plurality of slices comprised in the second medical image (IM2), the second slice based on degrees of slice similarity between image data comprised in the first slice and image data of individual slices of the second medical image (IM2).

10. Method according to any of claims 7 to 9,
wherein the step of determining (405) a dissimilarity between the extracted first slice and the extracted second slice comprises:
generating (501) a first feature vector (fv1) based on image data comprised in the first slice,
generating (502) a second input vector (fv2) based on image data comprised in the second slice,
wherein the step of determining (S30) the image similarity comprises calculating (S33) a similarity value (LS) by evaluating a similarity metric based on the first feature vector (fv1) and the second feature vector (fv2), and
wherein, in the step of determining (303, 405, S40) the dissimilarity, a dissimilarity is determined based on a comparison of the calculated similarity value (LS) with a pre-determined threshold.

11. Method according to claim 10, wherein
- the step of calculating (S33) the similarity value (LS) is executed for a plurality of different locations in the first slice and in the second slice,
wherein the step of determining (303, 405, S40) the dissimilarity comprises identifying a certain location as dissimilar based on a comparison of the calculated similarity value (LS) with the pre-determined threshold.

12. Method according to any of the preceding claims, wherein
- the step of determining (S30) the image similarity between image data of the first medical image (IM1) and image data of the second medical image (IM2) comprises:
applying a trained machine learning algorithm on image data of the first and second medical images (IM1, IM2), the trained machine learning algorithm is adapted to determine image similarities between medical images, and
optionally, wherein the trained machine learning algorithm applies a learned metric to determine image similarities images data, the trained machine learning algorithm preferably comprising a deep metric learning network.

13. A computer-implemented device (101) for automated processing of medical images (IM1, IM2) to output alerts (A) for detected dissimilarities in medical images (IM1, IM2), the computer-implemented device (101) comprising:
one or more processing units (201),
a receiving unit (204) which is configured to receive one or more medical images captured by a medical imaging unit (108), and
a memory (202) coupled to the one or more processing units (201), the memory (202) comprising a module (103) configured to perform the method steps according to any one of claims 1 to 12.

14. A computer program product comprising machine readable instructions, that when executed by one or more processing units (201), cause the one or more processing units (201) to perform the method steps according to any one of claims 1 to 12.

15. A computer readable medium on which program code sections of a computer program are saved, the program code sections being loadable into and/or executable in a system (100) to make the system (100) execute the method steps according to any one of the claims 1 to 12 when the program code sections are executed in the system (100).
